# EUROPÄISCHE PATENTSCHRIFT

(11) **EP 2 407 129 B1**
(45) Veröffentlichungstag und Bekanntmachung des Hinweises auf die Patenterteilung: **23.01.2013**
(21) Anmeldenummer: 10007302.2
(22) Anmeldetag: 15.07.2010
(51) Int. Cl.: A61F 5/44, A61F 5/445, A61F 2/00

(54) **Chirurgischer Stomaverschluss mit integriertem Schutz vor parastomalen Hernien und Überwachung der terminalen Blutversorgung im Hohlorgan**
Surgical stoma closure with integrated prevention of parastomal hernias and monitoring of terminal blood supply in the hollow organ
Obturation stomacale chirurgicale dotée d'une protection intégrée contre des hernies para-stomacales et surveillance de l'alimentation terminale en sang de l'organe creux

(43) Veröffentlichungstag der Anmeldung: 18.01.2012
(73) Patentinhaber: A.M.I. Agency for Medical Innovations GmbH, 6800 Feldkirch (AT)
(72) Erfinder: Egle, Walter, 6842 Koblach (AT)
(74) Vertreter: Hofmann, Ralf U.

(56) Entgegenhaltungen:
- WO-A1-90/07311
- US-A- 4 210 132
- US-A- 4 241 735
- US-A- 4 351 322
- US-A- 4 854 316

## Beschreibung

Das chirurgische Anlegen von temporären oder aber dauerhaften Ostomien ist eine weltweit sehr häufig durchgeführte Operation am Menschen. Unter Ostomie versteht man das chirurgische Schaffen einer temporären oder aber dauerhaften Verbindung von einem Hohlorgan zur Körperoberfläche hin. In den meisten Fällen handelt es sich beim betroffenen Hohlorgan um den Darm, im Besonderen um den Dickdarm, wobei das Ende des Darmes durch die Bauchdecke nach außen geführt und mit der Haut vernäht wird. Bei einer solchen Verbindung von Darm zur Körperoberfläche hin spricht der Arzt von einem Enterostoma, einer Kolostomie, einer Ileostomie oder allgemein von einem Stoma. Die Operation geht mit dem Verlust der Kontinenz einher, d.h. der Patient ist nicht mehr in der Lage den Austritt von Darminhalt nach außen zu kontrollieren. Bekannt ist eine Vielzahl von Systemen zum Auffangen des Darminhaltes. Dabei handelt es sich meistens um Auffangbeutel, die mittels Klebeplatten an der Haut des Patienten befestigt werden, dabei die Darmaustrittstelle umfassen und sich der Darminhalt somit in den Beutel entleeren kann. Vielen Patienten fällt es schwer die dauernde Notwendigkeit eines solchen Beutels zu akzeptieren. Deshalb gab es schon in der Vergangenheit viele Überlegungen wie kontinente Versorgung von Enterostomien aussehen könnten, damit der Patient auf das Tragen eines Auffangbeutels verzichten und über den Zeitpunkt der Darmentleerung wieder selbst entscheiden kann.

US 2,542,233 zeigt eine Verschlusskappe, die mittels Bauchriemen auf das Enterostoma gedrückt wird und dieses somit abgedichtet wird.

In US 3,937,224 wird ein Verschlusssystem gezeigt, bei dem aufblasbare Ballone in das Darmlumen (d.h. in den Hohlraum des Darmes) eingeführt und aufgeblasen werden. Die im Darm liegenden aufgeblasenen Ballone sollen für eine Abdichtung nach außen sorgen.

US 4,241,735 zeigt verschiedene Ausführungsformen aufblasbarer Verschlusstopfen, die von außen in den Darm eingeführt werden, bis sie innerhalb der Bauchhöhle liegen und dort aufgeblasen werden. Damit wird der Darmdurchgang abgedichtet und ein Herausrutschen der eingeführten Verschlussstopfen verhindert.

Eine mögliche Weiterentwicklung des historischen "Erlanger Magnet Verschlusses" wird in US 4,210,132 gezeigt. Dabei wird der Stomaverschlussstopfen über verschieden angeordnete Magnete in Position gehalten.

US 4,351,322 zeigt wie über einen Formschluss zwischen Stomaverschlussstopfen und einem an der Innenwand der Bauchdecke implantiertem Ring, durch welchen der Darm nach außen geführt wird, ein sicherer Verschluss des Darmes erzielt werden kann. Dabei wird der Stomaverschlussstopfen in den Darm eingeführt, bis dessen Ende hinter den implantierten Ring geführt wird. Dann wird der im Verschlussstopfen integrierte Ballon aufgeblasen und der Darm von innen an den implantierten Ring gedrückt. Damit wird der Darmdurchgang verschlossen und der Stomaverschlussstopfen am Herausrutschen aus dem Darm gehindert.

Eine Vielzahl von technischen Möglichkeiten zum dichten Verschluss von Enterostomata ist bekannt. Medizinische Komplikationen - meistens entzündliche Reaktionen - im Stomabereich wurden mit all diesen Systemen beobachtet und haben eine weite Anerkennung dieser patientenfreundlichen Möglichkeiten verhindert.

Medizinische Komplikationen in Verbindung mit Verschlussstopfen, die in die Darmöffnung eingeschoben und durch Formveränderung abdichten und vor dem Herausrutschen gesichert werden, stehen auch sehr oft in Verbindung mit einer reduzierten Blutversorgung des Darmes bis zum Darmende hin, welches an die Haut angenäht ist. Ursache dafür ist Druck, welcher auf die Darmwand ausgeübt wird, um ein Herausrutschen der Verschlussstopfen aus dem Darm zu verhindern, aber auch der zusätzliche Druck, der auf das den Darm mit Blut versorgende Mesenterium (eine Gewebefalte, die entlang des Darmes läuft und diesen mit Blut versorgt) im Bereich der Durchtrittsstelle durch die Bauchdecke ausgeübt wird.

Aufgabe der Erfindung ist es, einen sicheren, dichten Stomaverschluss mittels Verschlussstopfen zur Verfügung zu stellen, bei welchem die Gefahren der verminderten Durchblutung des Darmes bis zum Darmende hin ausgeschaltet wird.

Ein weiteres, bekanntes, medizinisches Problem von Langzeitenterostomien ist die Ausbildung von sogenannten "parastomalen Hernien". Als parastomale Hernien werden Bauchwanddefekte bezeichnet, die sich neben dem Darmdurchgang durch die Bauchdecke ausbilden und dazu führen können, dass Darmschlingen durch den Defekt in der Bauchdecke nach außen bis unter die Haut gedrückt werden. Dies erfordert einen weiteren chirurgischen Eingriff zum Beheben des am Darmdurchgang entstandenen Bauchwanddefektes, der sogenannten parastomalen Hernie. Bei 70% der Kolostomien (Dickdarm wird durch die Bauchdecke nach außen geführt und mit der Haut vernäht) entwickeln Patienten über längere Zeit eine sogenannte parastomale Hernie, von denen 10 bis 20% chirurgisch behandelt werden müssen.

Wie die Ausbildung von parastomalen Hernien verhindert werden kann, wird in US 4,854,316 gezeigt. Dabei wird die Durchtrittsstelle des Darmes durch die Bauchdecke mittels einer Netzprothese verstärkt. Die Anwendung von sogenannten Netzprothesen zur Behebung von Weichteildefekten ist in der Chirurgie seit Jahrzehnten bekannt. Bei diesen Netzprothesen handelt es sich um textile Gewebestrukturen, die aus geeigneten, implantierfähigen, bioinerten Textilfasern, wie z. B. Polypropylen oder Polyester, erzeugt werden. In US 4,854,316 wird gezeigt, wie dieses Netz auf der Innenseite der Bauchdecke um den Darm gelegt und mit der Bauchdecke vernäht wird.

Eine weitere Aufgabe der Erfindung ist es, einen sicheren, dichten Stomaverschluss mittels Verschlussstopfen zur Verfügung zu stellen, welcher gleichzeitig auch das Auftreten von parastomalen Hernien sicher verhindert.

Erfindungsgemäß nach Anspruch 1 wird bei der medizinischen Einrichtung die Dichtheit des Stomaverschlusses durch ein Zusammenwirken von einem im Darm liegenden Stomaverschlussstopfen und einer ringförmigen Struktur, die an der Durchtrittsstelle des Darmes durch die Bauchdecke den Darm am ganzen Umfang umschließt, erreicht. Durch integrierte, anpassbare und drucküberwachte Ballone wird ein Überdruck auf die formschlüssig umfasste Darmwand vermieden und die intramurale (in der Darmwand verlaufende) Blutversorgung sichergestellt.

Erfindungsgemäß nach Anspruch 1 wird das Auftreten von parastomalen Hernien dadurch verhindert, dass die den Darm umfassende ringförmige Struktur mit einem implantierfähigen Netz mit zirkulärer Durchgangsöffnung für den Darm verbunden ist, welches an der Durchtrittsstelle des Darmes durch die Bauchdecke, mit derselben vernäht wird. Die Verstärkung von Weichteilgewebe, wie z.B. der Bauchdecke, mittels implantierfähigen, textilen Netzen ist bekannt. Neu ist die Kombination eines Netzes mit einer ringförmigen Struktur, die den Darm an der Durchtrittsstelle durch die Bauchdecke umfasst und die nach Anspruch 1 vorzugsweise aus einem nach innen wirkenden Ringballon besteht. Der Ringballon kann über einen, an anderer Stelle unter der Haut implantierten Zugangsport, welcher durch einen Katheter mit dem Ringballon verbunden ist, mittels Flüssigkeit befüllt und / oder entleert werden. Damit kann der freie Innendurchmesser des Ballons patientenspezifisch angepasst werden.

Erfindungsgemäß wird die arterielle Blutversorgung bis zum Darmende - welches durch die Bauchdecke des Patienten geführt und mit der Hautoberfläche vernäht wird - kontrolliert. Die geschieht durch eine Druckmessung in dem hydraulischen System des Stomaverschlussstopfens, welches die Darmwand formschlüssig an eine entsprechend geformte Anpressfläche drückt, z.B. die den Darm umschließende, ringförmige Struktur mit einer von außen ablesbaren Druckanzeige mit entsprechender Grenzwertanzeige. Ist Gefahr für die Durchgängigkeit der intramuralen (in der Darmwand verlaufenden) Blutversorgung durch zu hohen Anpressdruck auf die Darmwand gegeben, wird das auf der Druckanzeige angezeigt und durch die Anpassung des Füllvolumens im hydraulischen System, einerseits im Stomaverschlussstopfen selbst oder aber andererseits im Ringballon, kann der Anpressdruck auf die Darmwand entsprechend reduziert werden.

Um die mesenteriale Blutversorgung des Darmes beim Durchtritt durch die ringförmige Struktur mit daran befestigtem Netz nicht zu behindern, wird erfindungsgemäß die geöffnete, ringförmige Struktur in einem avaskulären Bereich des Mesenteriums durch dasselbe durchgeführt und anschließend werden die beiden Enden der geöffneten ringförmigen Struktur zusammengefügt und miteinander verschlossen. Dazu hinterleuchtet der Chirurg mit einer Lichtquelle das Mesenterium des Darmes, wählt einen Bereich, in dem keine Blutgefäße sichtbar sind, und macht genau an dieser Stelle ein Loch in das Mesenterium des Darmes, groß genug zum Durchführen der ringförmigen Struktur der Erfindung. Das Netz, welches an der ringförmigen Struktur befestigt ist, hat einen Schlitz mit erfindungsgemäß zwei überlappenden Netzteilen, welche nach dem Vernähen miteinander eine Öffnung (ein Fenster) freigeben, die groß genug ist, um das Mesenterium des Darmes durchzuführen, ohne dass es zur Behinderung des Blutflusses im Mesenterium kommt.

Erfindungsgemäß kann die Größe der Öffnung im Netz während der Operation vom Chirurgen mittels Schere angepasst werden.

Umfang, Form und Fläche des implantierbaren Netzes, welches an der den Darm umfassenden, ringförmigen Struktur angebracht ist, kann erfindungsgemäß intraoperativ vom Chirurgen, mittels Schere angepasst werden. Damit kann die anatomisch notwendige Verstärkung der Bauchdecke, je nach Position des Stomas, patientengerecht gewählt werden. Das an der ringförmigen Struktur - z.B. am Ringballon - befestigte Netz wird entsprechend groß geliefert und vom Chirurgen während der Operation mittels Schere in die gewünschte Form gebracht. In weiterer Folge und in den Zeichnungen ist das Netz in kreisförmiger Struktur abgebildet.

Erfindungsgemäß kann die den Darm an der Durchtrittsstelle durch die Bauchdecke verstärkende, ringförmige Struktur auch aus einem weichen, implantierfähigen, dauerelastischen Schaumstoff, z.B. Silikon oder Polyurethan, gefertigt sein, deren Innendurchmesser - z.B. durch einen angeschlossenen Seilzug, welcher über eine unter der Haut liegende Einrichtung betätigt wird - patientengerecht eingestellt werden kann.

Der Stomaverschlussstopfen kann erfindungsgemäß mit einem Entlüftungskanal versehen sein, welcher z.B. mit Aktivkohle - zur Beseitigung von Gerüchen - gefüllt sein kann. Der Aktivkohlefilter kann integrierter Bestandteil des Stomaverschlussstopfens oder aber auch auswechselbar, als Filterpatrone ausgeführt sein.

Der Stomaverschlussstopfen kann erfindungsgemäß zusätzlich mit einem Spülkanal versehen sein, welcher mit einem handelsüblichen Anschluss für eine Spritze ausgestattet ist. Der handelsübliche Anschluss für Spritzen ist mit einem Rückschlagventil ausgestattet. Dem Patienten ermöglicht der Spülkanal im Stomaverschlussstopfen in den Darm rechtzeitig vor der Entleerung mittels Spritze Spülflüssigkeit einzubringen, welche den Darminhalt aufweicht, wodurch die Entleerung des Darmes erleichtert wird. Erfindungsgemäß kann die ringförmige Struktur, welche den Darm an der Durchtrittsstelle durch die Bauchdecke umfasst, als Ringballon ausgeführt sein, welcher über ein unter der Haut implantiertes System betätigt wird und den Darmdurchgang durch Druck auf die Darmwand dicht verschließt. Zum Entleeren des Darmes betätigt der Patient das ebenfalls unter der Haut liegende Rückschlagventil, der Ringballon entleert sich und der Darmdurchgang wird zur Stuhlentleerung freigegen. Anschließend betätigt der Patient, den ebenfalls und der Haut liegenden, weichen Flüssigkeitsbehälter durch Druck von außen, die Flüssigkeit wird zurück in den Ringballon gedrückt und der Darmdurchgang verschlossen.

Erfindungsgemäß kann ein sicherer Stomaverschluss durch eine Kombination
- eines äußeren, befüllbaren Ringballons in Verbindung mit einem Stomaverschlussstopfen mit integriertem, befüllbaren Ballon und Drucküberwachung
- eines äußeren Ringes aus Schaumstoff in Verbindung mit einem Stomaverschlussstopfen mit integriertem, befüllbaren Ballon und Drucküberwachung
- eines äußeren, befüllbaren Ringballons in Verbindung mit einem Stomaverschlussstopfen aus weichem Schaumstoff mit integrierter Drucküberwachung
- eines äußeren Ringes aus Schaumstoff in Verbindung mit einem Stomaverschlussstopfen aus weichem Schaumstoff mit integrierter Drucküberwachung,
- einer äußeren, ringförmigen Struktur aus einem festen Körper, z.B. aus implantierfähigem Metall oder Kunststoff, mit einem Stomaverschlussstopfen aus weichem Schaumstoff mit integrierter Drucküberwachung,
- einer äußeren, ringförmigen Struktur aus einem festen Körper, z.B. aus implantierfähigem Metall oder Kunststoff, mit einem Stomaverschlussstopfen mit integriertem, befüllbaren Ballon und Drucküberwachung
erzielt werden.

Weitere Vorteile und Einzelheiten der Erfindung werden im Folgenden anhand der beiliegenden Zeichnungen erläutert.

Fig. 1 zeigt eine mögliche Ausführungsform der Einrichtung mit einem ringförmigen Ballon in der Verschlussphase des Stomas am Patienten.

Fig. 2 zeigt eine mögliche Ausführungsform der Einrichtung mit einem ringförmigen Schaumstoffring in der Verschlussphase des Stomas am Patienten.

Fig. 3 zeigt eine mögliche Ausführungsform der Einrichtung bei welcher der ringförmige Ballon über ein, unter der Haut des Patienten liegendes System bestehend aus Rückschlagventil und elastischem Flüssigkeitsreservoir, durch Druck von außen auf das Rückschlagventil geöffnet und durch Druck von außen auf das Flüssigkeitsreservoir geschlossen wird. Zur Überwachung der Druckverhältnisse auf die Darmwand ist eine Kombination dieser Form der Einrichtung mit dem Stomaverschlussstopfen der Fig. 1 ist denkbar.

Fig. 4 zeigt als mögliche Ausführungsform ein kreisrundes Netz, befestigt an einem, geschlossenen ringförmigen Ballon und die Durchführung des Darmes durch die Einrichtung.

Fig. 5 zeigt eine mögliche Ausführungsform eines Stomaverschlussstopfens mit integriertem Messbereich zum Messen des Anpressdruckes der Darmwand.

Fig. 6 zeigt eine mögliche Ausführungsform eines Stomaverschlussstopfens mit intergriertem, befüllbarem Ballon.

Fig. 7 zeigt eine Ausführungsform der Einrichtung, bei welcher das Netz zur Verhütung von parastomalen Hernien über die, den Darm umfassende Ringstruktur gezogen ist und zusätzlich eine zylindrische, geschlitzte, überlappende Verlängerung aufweist, durch die der Darm durchgezogen wird.

Fig. 8 zeigt eine weitere Ausführungsform des Stomaverschlussstopfens mit einem zusätzlich integrierten Spülkanal.

Die Figuren weisen unterschiedliche Maßstäbe auf.

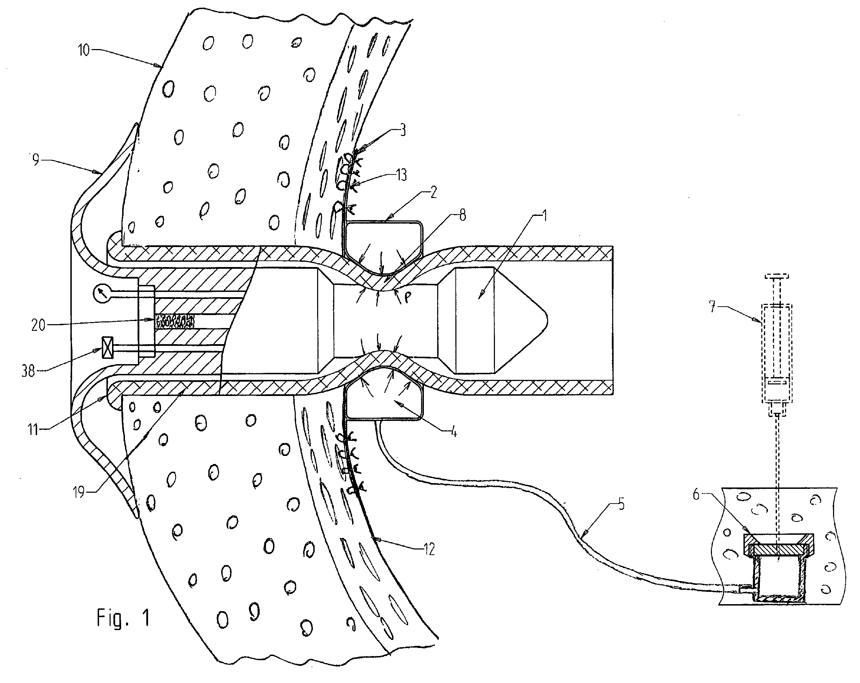

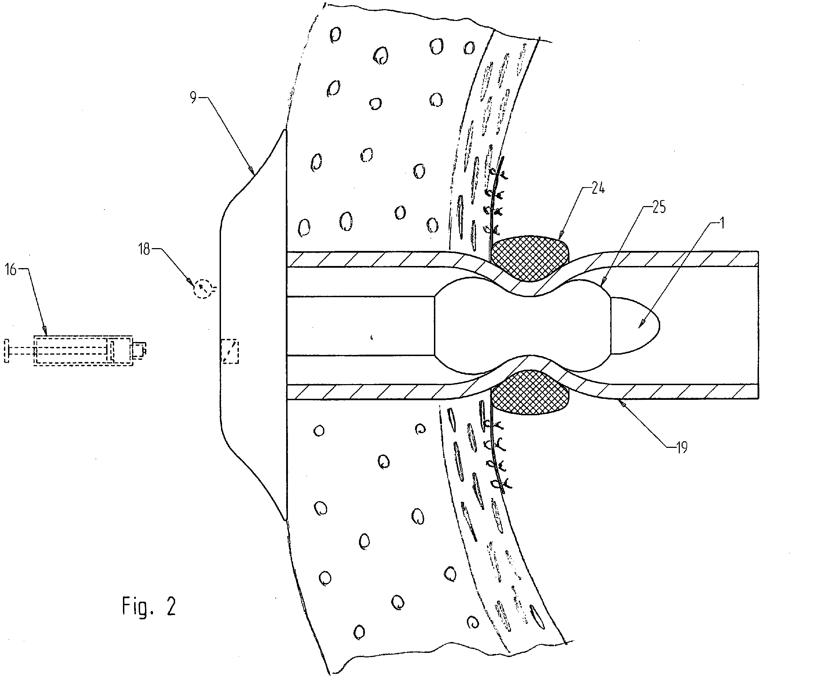

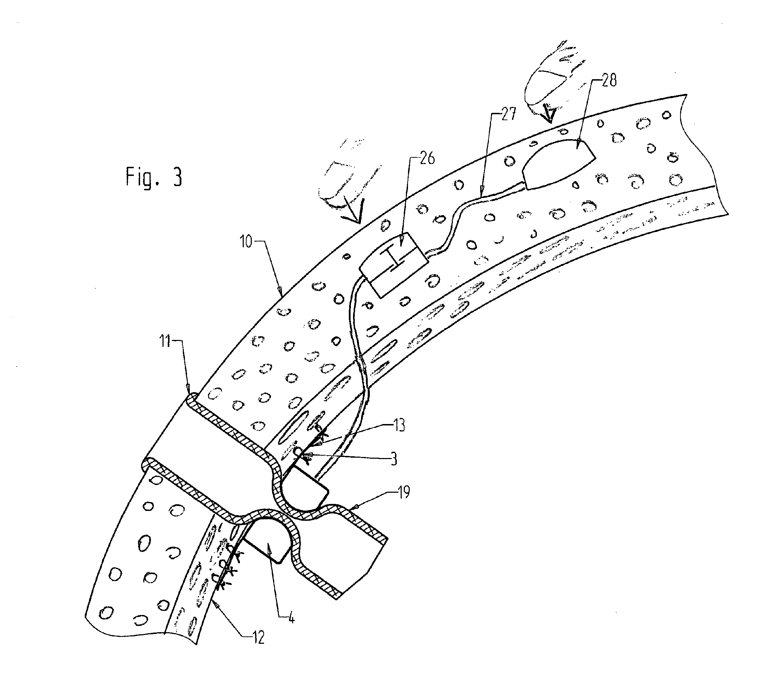

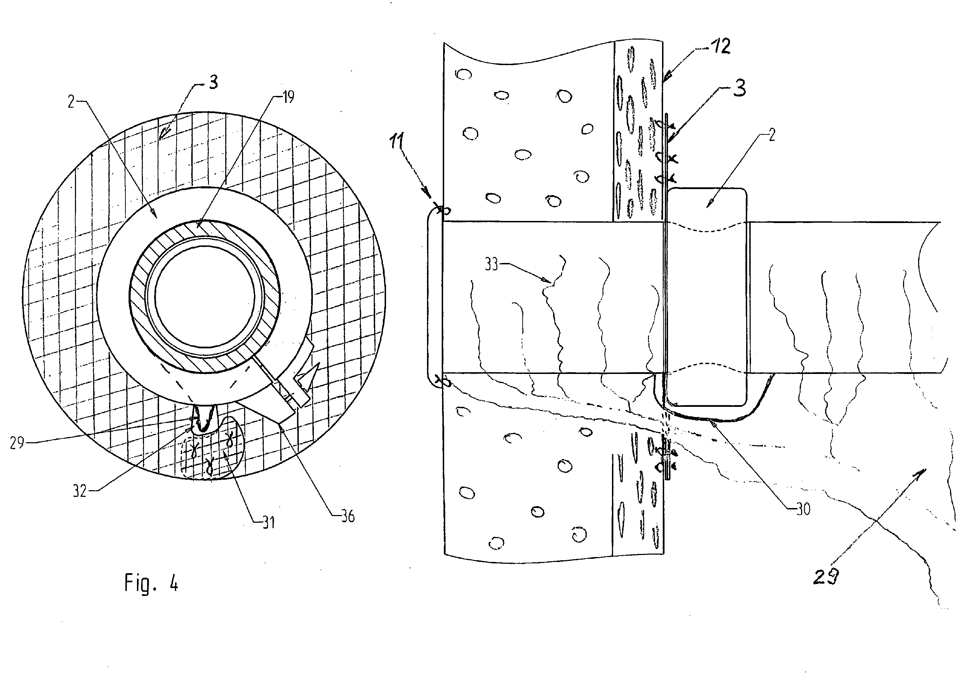

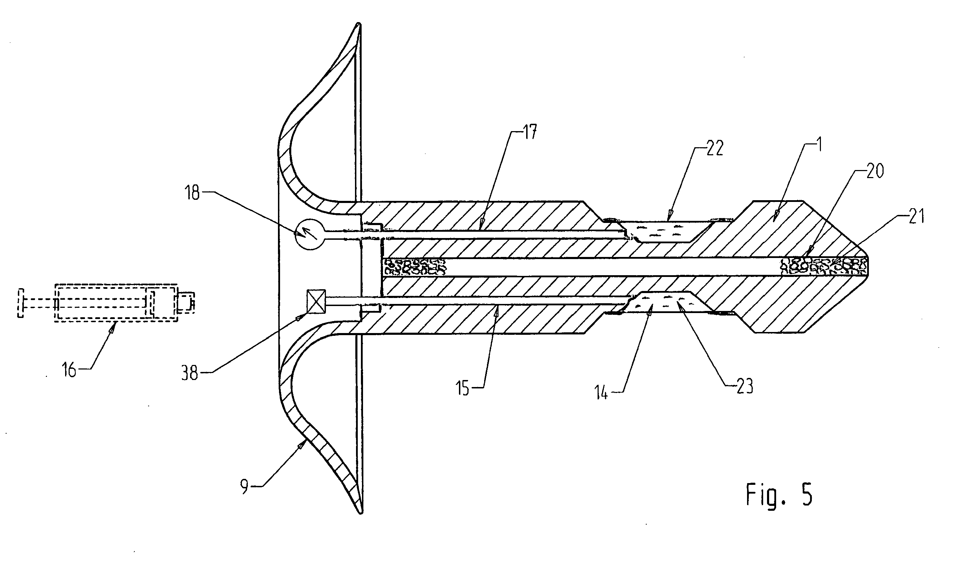

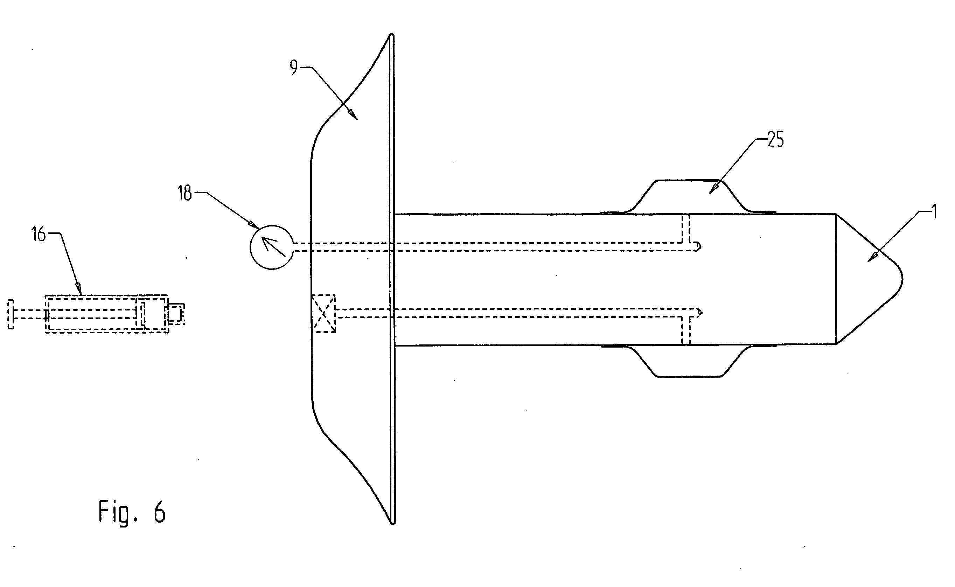

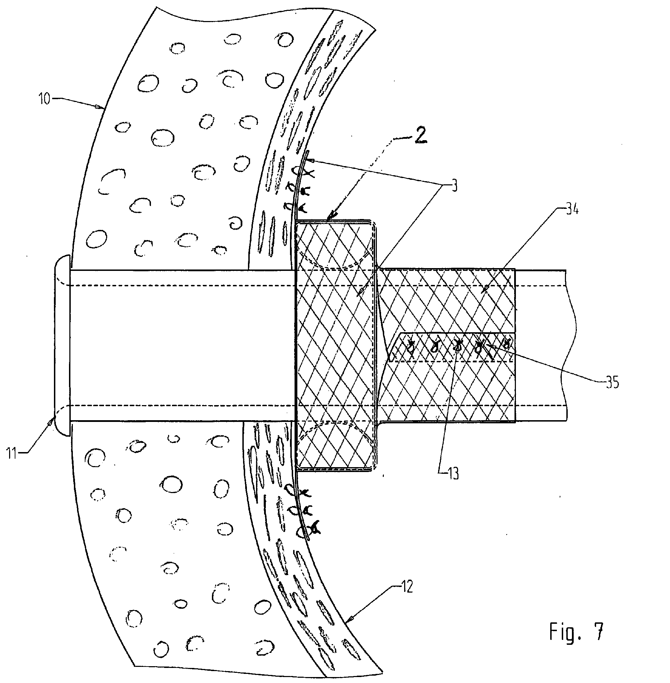

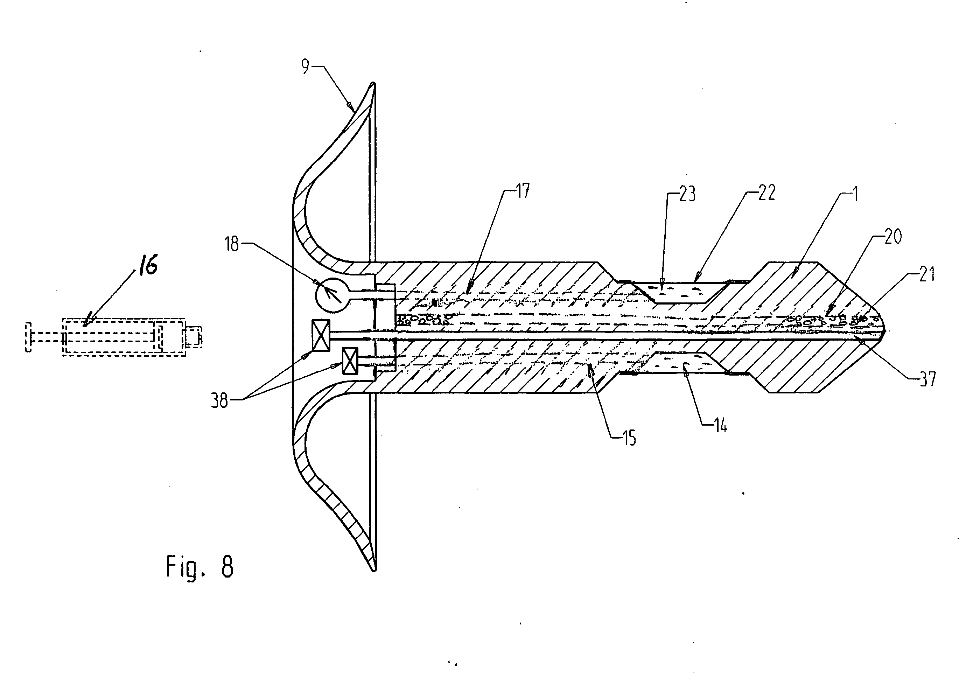

Eine nicht vollständige Auswahl von Ausführungsbeispielen der medizinischen Einrichtung gemäß der Erfindung wird in den Figuren 1 bis 8 dargestellt. Die Einrichtung besteht aus einem Stomaverschlussstopfen 1 und einer, den Darm umfassenden ringförmigen Struktur 2 mit Verschlusseinrichtung 36, an der eine Flächenstruktur aus implantierfähigem, textilem Netz 3 befestigt ist. Die den Darm umfassende ringförmige Struktur kann als befüllbarer Ringballon 4 ausgeführt sein, welcher über einen durch Katheter 5 mit einem an anderer Stelle unter der Haut implantierten Füllport 6 verbunden ist und mittels Spritze mit Portnadel 7 mit Flüssigkeit, z.B. Kochsalzlösung, befüllt und entleert werden kann. Das an der ringförmigen Struktur 2 umfänglich befestigte, flächige Netz 3, wird an der inneren Faszie 12 der Bauchdecke mit genügend Einzelknopfnähten 13 aus nicht resorbierbarem Nahtmaterial angenäht.

Der Ringballon 4 wird über den Füllport 6 mit Flüssigkeit befüllt und der nach innen ausdehnende, zirkuläre Ballon drückt von außen auf die Darmwand und sorgt für eine Einengung 8 des Darmes. Zum Abdichten der Darmöffnung zur Körperoberfläche hin, wird nun der Stomaverschlussstopfen 1 von außen in den Darm hineingeschoben, bis der Flansch 9 auf der Haut 10 des Patienten liegt.

Der Flansch 9 kann zusätzlich mittels Klebeband oder Verband auf der Haut 10 des Patienten gesichert werden. Handelsübliche, dünne Verbandklebefolien bieten sich dafür an.

Der Flansch 9 ist derart ausgeführt, dass das an der Haut angenähte Ende des Darmes 11 vom Flansch 9 vollkommen abgedeckt wird.

Der Stomaverschlussstopfen 1 ist mit einer Druckmesseinrichtung zum Erfassen des Anpressdruckes auf die Darmwand im Bereich des Formschlusses zwischen ringförmiger Struktur 2 und Stomaverschlussstopfen 1, ausgestattet. Die Druckmesseinrichtung besteht aus dem mit Flüssigkeit gefüllten, zylindrischen Messteil 14, welches über einen Kanal 15 mittels Spritze mit Schraubanschluss 16 befüllt und entlüftet werden kann. Ein Messkanal 17 verbindet das Messteil 14 mit einer Druckanzeige 18. Die Druckanzeige 18 ist vom Patienten einsehbar und verfügt über eine Grenzwertmarkierung, z.B. roter Bereich, welcher dem Patienten signalisiert, dass der Anpressdruck auf die Darmwand 19 zu hoch ist und eine sichere Durchblutung des Darmes bis zum Darmende 11 nicht mehr gewährleistet ist. Durch eine Reduzierung des Füllvolumens im Ringballon 4 kann der Anpressdruck p (siehe Fig. 1) auf die Darmwand 19 verringert werden. Das zylindrische Messteil 14 des Stomaverschlussstopfens 1, besteht z.B. aus einem dünnen, zylindrischen, elastischen Schlauchstück 22, welches auf dem Stomaverschlussstopfen 1 aufgeklebt ist und einen mit Flüssigkeit gefüllten, auf Außendruck reagierenden Hohlraum 23 schafft.

In einer weiteren Ausführungsform der Erfindung, besteht die den Darm umfassende ringförmige Struktur aus einem, z.B. über Seilzug im Durchmesser verstellbaren Ring 24 aus implantierfähigem, weichem Schaumstoff und der Stomaverschlussstopfen 1 verfügt über einen integrierten, befüllbaren zylindrischem Ballon 25. Der zum sicheren Abdichten des Stomas benötigte Formschluss zwischen dem Ring 24 aus Schaumstoff und dem eingeführten Stomaverschlussstopfen 1 wird bei dieser Ausführungsform durch das Befüllen des zylindrischen Ballons 25 erzielt, wobei das Befüllen des Ballons 25 auch nach Einführung des Stomaverschlussstopfen 1 in den Darm, mittels Spritze 16 erfolgen kann. Dabei wird die Druckanzeige 18 beobachtet um sicherzustellen, dass kein zu hoher Anpressdruck auf die Darmwand 19 deren Durchblutung stoppt.

Der Stomaverschlussstopfen 1 kann mit einem Entlüftungskanal 20 ausgestattet sein, welcher mit Aktivkohle 21 gefüllt ist.

In einer weiteren Ausführungsform der Erfindung, kann der Ringballon 4 soweit geschlossen werden, dass der Durchgang im zirkulär umschlossenen Darm 19 verschlossen wird. Zum Entleeren des Darmes drückt der Patient auf ein an anderer Stelle unter der Haut implantiertes Ventil 26. Dadurch fließt die Systemflüssigkeit aus dem expandierten, unter Druck stehenden Ringballon 4 über den Katheter 27 in ein, ebenfalls unter der Haut implantiertes, leeres, elastisches Flüssigkeitsreservoirs 28. Zum Schließen des Ringballons 4 drückt der Patient auf das unter der Haut liegende, elastische Flüssigkeitsreservoir 28, dabei wird die Systemflüssigkeit durch das Ventil 26 zurück in den Ringballon 4 gedrückt. Um den Anpressdruck auf die Darmwand 19 in geschlossenem Zustand des Ringballons 4 zu kontrollieren, kann auch diese Ausführungsform der Erfindung mit einem Stomaverschlussstopfen 1, mit integriertem Messteil 14 kombiniert werden.

Neben dem Aufrechterhalten der intramuralen (in der Darmwand verlaufenden) Durchblutung des Darmes ist es ebenso wichtig, die mesenteriale Blutversorgung des Darmes bis zu dessen Ende 11 sicherzustellen. Das Mesenterium 29 ist eine Gewebefalte, die in Längsrichtung über die ganze Länge am Darm angewachsen ist und diesen neben anderen Funktionen mit Blut versorgt. Um dies zu erreichen wird die ringförmige Struktur 2 durch ein vom Chirurgen in einem avaskulären Bereich des Mesenteriums 29 geschaffenen Fenster 30 geführt. Das mit der ringförmigen Struktur 2 verbundene Netz 3 ist geschlitzt und verfügt über zwei sich überlappende Enden 31, die vom Chirurgen miteinander, überlappend an der inneren Faszie 12 der Bauchdecke vernäht werden. Dabei entsteht eine Lücke 32 im Netz 3, durch welche das Mesenterium 29 des Darmes 19 durch das Netz 3 durchgeführt werden kann, ohne dass es zu einer Behinderung der Durchblutung in den mesenterialen Gefäßen 33 kommt.

In einem weiteren Ausführungsbeispiel der medizinischen Einrichtung zum Verhindern von parastomalen Hernien, wird die ringförmige Struktur 2, die den Darm an der Durchtrittsstelle durch die Bauchdecke umfänglich umfasst, von der implantierfähigen, textilen Netzstruktur 3 umhüllt und durch eine zylindrische Verlängerung 34 derselben ergänzt. Die zylindrische Verlängerung 34 des Netzes 3 ist zum Durchführen des Darmes in Längsrichtung überlappend, geschlitzt ausgeführt. Der zylindrisch, überlappende Teil 35 des Netzes 3 wird nach dem Durchführen des Darmes durch Einzelknopfnähte 13 miteinander vernäht.

In einem weiteren Ausführungsbeispiel des Stomaverschlussstopfen 1, ist dieser mit einem zusätzlichen Spülkanal 37 versehen. Der Spülkanal ist auf der Flanschseite 9 mit einem handelsüblichen Anschluss 38 für Spritzen 16 ausgeführt. Der handelsübliche Anschluss 38 für Spritzen ist mit einem integrierten Rückschlagventil ausgestattet und verschließt den Spülkanal, wenn keine Spritze 16 angeschlossen ist.

### Legende zu den Hinweisziffern:

- 1: Stomaverschlussstopfen
- 2: Ringförmige Struktur
- 3: Netz
- 4: Ringballon
- 5: Katheter
- 6: Füllport
- 7: Spritze mit Portnadel
- 8: Einengung des Darmes
- 9: Flansch
- 10: Haut des Patienten
- 11: Ende des Darmes
- 12: Faszie
- 13: Einzelknopfnähte
- 14: Messteil
- 15: Kanal
- 16: Spritze mit Schraubanschluss
- 17: Messkanal
- 18: Druckanzeige
- 19: Darmwand
- 20: Entlüftungskanal
- 21: Aktivkohle
- 22: Elastisches, weiches Schlauchstück
- 23: Hohlraum
- 24: Verstellbarer Ring
- 25: Zylindrischer Ballon
- 26: Ventil
- 27: Katheter
- 28: Flüssigkeitsreservoir
- 29: Mesenterium des Darmes
- 30: Fenster im Mesenterium
- 31: Überlappende Enden vom Netz
- 32: Lücke im Netz
- 33: Mesenteriale Gefäße
- 34: Zylindrische Verlängerung des Netzes
- 35: Zylindrisch überlappender Teil des Netzes
- 36: Verschlusseinrichtung der ringförmigen Struktur
- 37: Spülkanal
- 38: Spritzenanschluss mit Rückschlagventil

## Patentansprüche

1. Medizinische Einrichtung zum Verschließen von chirurgisch geschaffenen Öffnungen von Hohlorganen zur Körperoberfläche hin, insbesondere von Kolostomien (11), bei welcher das Abdichten des Darmes zur Körperoberfläche hin durch ein Zusammenwirken einer, den Darm umfassenden ringförmigen Struktur (2) mit Verschlusseinrichtung (36) und einem, in den Darm eingeführten Stomaverschlussstopfen (1) erfolgt, die einen Formschluss bilden, welcher den Darmausgang verschließt, **dadurch gekennzeichnet, dass** an der den Darm umfassenden, ringförmigen Struktur (2) umfänglich ein flächiges, textiles Netz (3), fest verbunden, angebracht ist, womit die ringförmige Struktur (2) an der Durchtrittsstelle des Darmes sicher an der inneren Faszie (12) der Bauchdecke, z.B. mittels Einzelknopfnähten (13), angenäht werden kann und dadurch das Auftreten von parastomalen Hernien verhindert wird.

2. Medizinische Einrichtung nach Anspruch 1 **dadurch gekennzeichnet, dass** der Anpressdruck auf die Darmwand im Bereich des Formschlusses zwischen der den Darm umfassenden ringförmigen Struktur (2) und dem Stomaverschlussstopfen (1) über eine im Stomaverschlussstopfen integrierte Druckanzeige (18) überwacht wird.

3. Medizinische Einrichtung nach Anspruch 1 **dadurch gekennzeichnet, dass** die ringförmige Struktur (2) als befüllbarer Ringballon (4) ausgeführt ist, dessen Füllvolumen über einen unter der Haut implantierten Füllport (6) angepasst werden kann.

4. Medizinische Einrichtung nach Anspruch 1 **dadurch gekennzeichnet, dass** die ringförmige Struktur (2) aus einem verstellbaren Ring (24) aus weichem, implantierfähigem Schaumstoff besteht und der Stomaverschlussstopfen mit einem zylindrischen, befüllbaren Ballon (25) ausgestattet ist und damit der benötigte Formschluss zwischen äußerer ringförmigen Struktur (2) und dem Stomaverschlussstopfen (1) durch Befüllen des zylindrischen Ballon (25) hergestellt wird.

5. Medizinische Einrichtung nach Anspruch 1 **dadurch gekennzeichnet, dass** das, an der ringförmigen Struktur (2) fest angebrachte, flächige, aus implantierfähigen textilen Fasern gefertigte Netz (3) die ringförmige Struktur an deren Außenumfang umhüllt und mit einer, in Längsrichtung geschlitzten zylindrischen Verlängerung (34) versehen ist, durch welche der zur Körperoberfläche verlaufende Darm durchgeführt wird.

6. Medizinische Einrichtung nach Anspruch 1 **dadurch gekennzeichnet, dass** das flächige, mit der ringförmigen Struktur fest verbundene Netz (3) zum Herumführen um den Darm geschlitzt ist und die beiden sich überlappenden Enden (31) des Netzes (3) beim Zusammennähen durch den Chirurgen eine Lücke (32) ergeben, durch welche das Mesenterium (29) des Darmes durchgeführt werden kann.

7. Medizinische Einrichtung nach Anspruch 1 **dadurch gekennzeichnet, dass** die ringförmige Struktur (2) als Ringballon (4) ausgeführt ist, welcher über ein unter der Haut implantiertes System, bestehend aus Katheter (27), aus Ventil (26) und aus Flüssigkeitsreservoir (28) vom Patienten mittels Druck auf die Haut, geöffnet bzw. geschlossen werden kann.

8. Medizinische Einrichtung nach Anspruch 1 **dadurch gekennzeichnet**, das der Stomaverschlussstopfen (1) mit einem Flansch (9) ausgestattet ist, welcher eine vollständige Abdeckung des offenen Darmendes (11) ermöglicht und auf der Haut (10) des Patienten mit Klebeverband gesichert werden kann.

9. Medizinische Einrichtung nach Anspruch 1 **dadurch gekennzeichnet, dass** der Stomaverschlussstopfen (1) mit einem Entlüftungskanal (20) versehen ist, welcher z.B. zur Verhinderung von Geruchsbelästigung mit Aktivkohle (21) gefüllt ist.

10. Medizinische Einrichtung nach Anspruch 1 **dadurch gekennzeichnet, dass** der Stomaverschlussstopfen (1) zusätzlich mit einem Spülkanal (37) ausgestattet ist, welcher durch einen handelsüblichen Spritzenanschluss mit Rückschlagventil (38) verschlossen ist.

## Claims

1. A medical device for the closing of surgically created openings from hollow organs to the body surface, in particular of colostomies (11), with which the sealing of the intestine with respect to the body surface takes place through a cooperation of an intestine-embracing, annular structure (2) with closing device (36) and a stoma closing plug (1) inserted into the intestine, these forming a form lock which closes the intestinal outlet, **characterised in that** to the intestine-embracing, annular structure (2) there is circumferentially attached a planar, textile mesh (3) in a securely connected manner, whereby the annular structure (2) can at the emergence site of the intestine be securely sutured to the inner fascia (12) of the abdominal wall, e.g. by means of simple interrupted sutures (13), and the occurrence of parastomal hernias is thereby avoided.

2. A medical device according to claim 1, **characterised in that** the contact pressure on the intestinal wall is monitored in the region of the form lock between the intestine-embracing, annular structure (2) and the stoma closing plug (1) via a pressure indicator (18) integrated in the stoma closing plug.

3. A medical device according to claim 1, **characterised in that** the annular structure (2) is in the form of a fillable annular balloon (4) whose filling volume can be adapted via a filling port (6) implanted below the skin.

4. A medical device according to claim 1, **characterised in that** the annular structure (2) consists of an adjustable ring (24) of soft, implantable expanded plastic and the stoma closing plug is furnished with a cylindrical, fillable balloon (25) and thereby the required form lock between outer, annular structure (2) and the stoma closing plug (1) is produced by filling of the cylindrical balloon (25).

5. A medical device according to claim 1, **characterised in that** the planar mesh (3), securely attached to the annular structure (2) and manufactured from implantable, textile fibres, encases the annular structure at its outer circumference and is provided with a cylindrical prolongation (34), slit in the longitudinal direction, through which there is guided the intestine running to the body surface.

6. A medical device according to claim 1, **characterised in that** the planar mesh (3) securely connected to the annular structure is slit for guiding around the intestine and the two overlapping ends (31) of the mesh (3) result in a gap (32) upon suturing together by the surgeon, through which gap the mesentery (29) of the intestine can be guided.

7. A medical device according to claim 1, **characterised in that** the annular structure (2) is in the form of an annular balloon (4) which can be opened and closed by the patient, by means of pressure on the skin, via a system which is implanted below the skin and which consists of catheter (27), valve (26) and fluid reservoir (28).

8. A medical device according to claim 1, **characterised in that** the stoma closing plug (1) is furnished with a flange (9) which enables complete covering of the open intestinal outlet (11) and which can be secured on the skin (10) of the patient using adhesive dressing.

9. A medical device according to claim 1, **characterized in that** the stoma closing plug (1) is provided with a ventilation channel (20) which is e.g. filled with activated carbon (21) to prevent odorous annoyance.

10. A medical device according to claim 1, **characterised in that** the stoma closing plug (1) is additionally furnished with a flushing channel (37) closed by a commercial injection connection with non-return valve (38)

## Revendications

1. Dispositif médical pour permettre de refermer des ouvertures effectuées chirurgicalement d'organes creux vers la surface du corps, en particulier de colostomies (11) dans lequel l'étanchéité de l'intestin vers la surface du corps est réalisée par la coopération d'une structure annulaire (2) entourant l'intestin munie d'un dispositif de fermeture (36) et d'un bouchon de fermeture de la stomie (1) introduit dans l'intestin, qui définissent une liaison par la forme qui ferme la sortie de l'intestin,
**caractérisé en ce qu'**
un treillis textile plan (3) est appliqué à la périphérie de la structure annulaire (2) entourant l'intestin, en étant relié solidairement à celle-ci de sorte que la structure annulaire (2) puisse être suturée de façon sûre, au niveau de l'ouverture de l'intestin, sur le fascia (12) de la paroi abdominale, par exemple à l'aide de sutures point par point (13), et que l'on puisse ainsi empêcher l'apparition de hernies para-stomacales.

2. Dispositif médical conforme à la revendication 1,
**caractérisé en ce que**
la force de serrage sur la paroi de l'intestin dans la zone de la liaison par la forme entre la structure annulaire (2) entourant l'intestin et le bouchon de fermeture (1) de la stomie est surveillée par l'intermédiaire d'un indicateur de pression (18) intégré dans le bouchon de fermeture de la stomie.

3. Dispositif médical conforme à la revendication 1,
**caractérisé en ce que**
la structure annulaire (2) est réalisée sous la forme d'un ballon annulaire (4) pouvant être rempli dont le volume de remplissage peut être adapté par l'intermédiaire d'un orifice de remplissage (6) implanté sous la peau.

4. Dispositif médical conforme à la revendication 1,
**caractérisé en ce que**
la structure annulaire (2) est constituée par un anneau réglable (24) en un matériau alvéolaire souple pouvant être implanté, et le bouchon de fermeture de la stomie est équipé d'un ballon cylindrique (25) pouvant être rempli, et la liaison par la forme nécessaire entre la structure annulaire externe (2) et le bouchon de fermeture de la stomie (1) est ainsi obtenue par remplissage du ballon cylindrique (25).

5. Dispositif médical conforme à la revendication 1,
**caractérisé en ce que**
le treillis plan (3) constitué de fibres textiles pouvant être implantées et appliqué solidairement sur la structure annulaire (2) entoure cette structure annulaire sur sa périphérie externe et est équipé d'un prolongement cylindrique (34) fendu en direction longitudinale au travers duquel peut être passé l'intestin s'étendant vers la surface du corps.

6. Dispositif médical conforme à la revendication 1,
**caractérisé en ce que**
le treillis plan (3) relié solidairement à la structure annulaire (2) est fendu pour lui permettre d'entourer l'intestin, et les deux extrémités (31) qui se chevauchent de ce treillis (3) forment, lors de leur suture par le chirurgien une lacune (32) par laquelle peut être introduit le mésentère (29) de l'intestin.

7. Dispositif médical conforme à la revendication 1,
**caractérisé en ce que**
la structure annulaire (2) est réalisée sous la forme d'un ballon annulaire (4) qui peut être ouvert ou fermé par le patient par l'intermédiaire d'un système implanté sous la peau constitué d'un cathéter (27) d'une soupape (26) et d'un réservoir de liquide (28), en exerçant une pression sur la peau.

8. Dispositif médical conforme à la revendication 1,
**caractérisé en ce que**
le bouchon de fermeture de la stomie (1) est équipé d'une bride (9) qui permet un recouvrement total de l'extrémité ouverte (11) de l'intestin et peut être bloqué sur la peau (10) du patient par un bandage adhésif.

9. Dispositif médical conforme à la revendication 1,
**caractérisé en ce que**
le bouchon de fermeture de la stomie (1) est équipé d'un canal de ventilation (20) qui est rempli de charbon actif (21) notamment pour empêcher les mauvaises odeurs.

10. Dispositif médical conforme à la revendication 1,
**caractérisé en ce que**
le bouchon de fermeture de la stomie (1) est en outre équipé d'un canal de rinçage (37) qui est fermé par un raccord de pulvérisation du commerce équipé d'une soupape anti-retour (38).
